# EUROPEAN PATENT APPLICATION

(11) **EP 4 560 010 A1**
(43) Date of publication of application: **28.05.2025**
(21) Application number: 23843338.7
(22) Date of filing: 18.07.2023
(51) Int. Cl.: C12N 5/071, G01N 33/50, C12Q 1/70, C12Q 1/6883

(54) **COVID-19 AND RESPIRATORY DISEASE VIRUS INFECTION MODELS USING LUNG ORGANOIDS**

(30) Priority: 20.07.2022 KR 20220089724; 27.06.2023 KR 20230082287
(71) Applicant: The Catholic University Of Korea Industry-Academic Cooperation Foundation, Seocho-gu Seoul 06591 (KR)
(72) Inventor: KIM, Sung Won, Seoul 06001 (KR); JO, Ayoung, Seoul 07075 (KR); LIM, Min Jae, Pyeongtaek-si, Gyeonggi-do 17880 (KR)
(74) Representative: Nederlandsch Octrooibureau
(86) International application number: PCT/KR2023/010322
(87) International publication number: WO 2024/019492

(57) **Abstract**

The present invention relates to a COVID-19 infection model using alveolar organoids. Respiratory virus-infected alveolar organoids produced according to the present invention are expected to be usefully used in preclinical or clinical drug screening and the like for the development of therapeutic agents for SARS-CoV-2 infection.

## Description

### [Technical Field]

The present invention relates to a COVID-19 and respiratory disease virus infection model, and the like using alveolar organoids.

The present invention claims priority to and the benefit of Korean Patent Application Nos. 10-2022-0089724 and 10-2023-0082287 filed in the Korean Intellectual Property Office on July 20, 2022 and June 27, 2023, respectively, and all the contents disclosed in the specifications and drawings of those applications are incorporated in this application.

### [Background Art]

Coronaviruses are positive-sense single-stranded (+ssRNA) viruses, and are characterized by a crown-shaped envelope surrounded by spike proteins. Coronaviruses are known to be capable of inducing respiratory, intestinal, liver, digestive and neurological diseases in animals such as mammals such as cattle, camels, cats, rats, bats, and humans and birds.

Coronaviridae (family Coronaviridae) includes the genera Alphacoronavirus (α-CoV, alpha-CoV, alpha-coronavirus), the genera Betacoronavirus (β-CoV, beta-CoV, beta-coronavirus), the genera Gammacoronavirus (γ-CoV, gamma-CoV, gammacoronavirus), and the genera Deltacoronavirus (δ-CoV, delta-CoV, delta-coronavirus). Alpha-CoV includes HCoV-229E, HCoV-NL63, and the like. Beta-CoV includes Bat-SARS-like(SL)-ZC45, Bat-SL ZXC21, SARS-CoV, MERS-CoV, HCoV-OC43, HKU-1, MHV-A59, SARS-CoV-2, and the like.

SARS-CoV-2 was first reported to have emerged in Wuhan, China in December 2019. As of July 2020, the number of people infected with SARS-CoV-2 worldwide exceeded 12,500,553, of which 560,271 died due to the infection. Upon infection with SARS-CoV-2, the most typical clinical symptoms are lower respiratory symptoms (dry cough), high fever, and dyspnea. According to Chinese researchers who analyzed 72,314 people confirmed to be infected, about 14% were severe cases with dyspnea (oxygen saturation below 93%, pulmonary infiltration 24%), 5% showed life-threatening symptoms showing respiratory failure, organ failure and dysfunction, and septic shock, and 81% showed mild symptoms with or without pneumonia. SARS-CoV-2 is primarily transmitted through the upper respiratory tract, and its symptoms range from mild severity (fever, cough, sore throat, anosmia or ageusia, and the like) to extreme severity (pneumonia, death, and the like).

Efforts are underway worldwide to find active ingredients that demonstrate antiviral effects against the above coronaviruses and therapeutic or prophylactic effects against coronavirus infections and coronavirus-related diseases or conditions. Therefore, there is a need for rapidly exploring candidate substances and subjecting them to clinical trials, and for this purpose, there is a need for an ex vivo model capable of mimicking human infection in terms of the antiviral pathway as well as the host immune response.

### [Disclosure]

### [Technical Problem]

The present inventors treated an alveolar organoid with a respiratory virus to infect the alveolar organoid with the respiratory virus and establish a respiratory viral infection model using the alveolar organoid, thereby completing the present invention.

It is an object of the present invention to provide a method for producing a respiratory virus-infected alveolar organoid, the method comprising infecting an alveolar organoid with a respiratory virus.

It is another object of the present invention to provide a respiratory virus-infected alveolar organoid produced by the method for producing a respiratory virus-infected alveolar organoid.

It is still another object of the present invention to provide a method for screening a therapeutic agent for respiratory viral infections, the method comprising treating the respiratory virus-infected alveolar organoid according to the present invention with a candidate substance for a therapeutic agent for respiratory viral infections.

However, the technical problems which the present invention intends to solve are not limited to the technical problems that have been mentioned above, and other technical problems that have not been mentioned will be clearly understood by a person with ordinary skill in the art to which the present invention pertains from the following description.

### [Technical Solution]

To achieve the purpose of the present invention, the present invention provides a method for producing a respiratory virus-infected alveolar organoid, the method comprising infecting an alveolar organoid with a respiratory virus.

In addition, the present invention provides a respiratory virus-infected alveolar organoid produced by the method for producing a respiratory virus-infected alveolar organoid.

In an embodiment of the present invention, the respiratory virus may be infected so that a multiplicity of infection (MOI) is 0.001 to 10, but is not limited thereto.

In another embodiment of the present invention, the respiratory virus may be one or more selected from the group consisting of an adenovirus, metapneumovirus, rhinovirus, parainfluenza virus, influenza virus, respiratory syncytial virus, and coronavirus, but is not limited thereto.

In still another embodiment of the present invention, the respiratory virus may be an influenza virus (PR8) or severe acute respiratory syndrome coronavirus 2, but is not limited thereto.

In yet another embodiment of the present invention, when the respiratory virus is severe acute respiratory syndrome coronavirus 2, the respiratory virus-infected alveolar organoid may have decreased expression of one or more proteins selected from the group consisting of angiotensin-converting enzyme 2 (ACE2), transmembrane serine protease 2 (TMPRESS2), alveolar type 1 (AT1), alveolar type 2 (AT2), and surfactant protein C (SFTPC) proteins compared to non-infected alveolar organoids, but is not limited thereto.

In yet another embodiment of the present invention, when the respiratory virus is severe acute respiratory syndrome coronavirus 2, the respiratory virus-infected alveolar organoid may have increased expression of one or more proteins selected from the group consisting of interferon-alpha/beta receptor (IFNR α/β), signal transducer and activator of transcription 1 (STAT1), and signal transducer and activator of transcription 2 (STAT2) proteins compared to non-infected alveolar organoids, but is not limited thereto.

In addition, the present invention provides a method for screening a therapeutic agent for respiratory viral infections, the method comprising treating the respiratory virus-infected alveolar organoid according to the present invention with a candidate substance for a therapeutic agent for respiratory viral infections.

In addition, the present invention provides a use of the respiratory virus-infected alveolar organoid according to the present invention for screening a therapeutic agent for respiratory viral infections.

### [Advantageous Effects]

Respiratory virus-infected alveolar organoids produced according to the present invention are expected to be effectively used in preclinical or clinical drug screening and the like for the development of therapeutic agents for respiratory viral infections.

### [Description of Drawings]

FIG. 1 is a view illustrating the results of measuring the amount of virus released into the culture medium from COVID-19-infected alveolar organoids at 3 hours, 1 day, 2 days, and 3 days after injection of the COVID-19 virus, according to the amount of COVID-19 virus injected into the alveolar organoids.
FIG. 2 is a view illustrating the results of confirming the expression of virus-specific factors, spike and nucleocapsid, in COVID-19-infected alveolar organoids.
FIG. 3 is a view illustrating the results of confirming the expression of nucleocapsid in COVID-19-infected alveolar organoids through fluorescent staining (inf: infection).
FIG. 4 is a view illustrating the results of confirming the expression of ACE2 and TMPRESS2 in COVID-19-infected alveolar organoids through fluorescent staining.
FIG. 5 is a view illustrating the results of confirming the expression of nucleocapsid, ACE2, TMPRESS2, HT1-56 (AT1), HT2-280 (AT2), SPC (SFTPC), IFNRα/β, STAT1, and STAT2 in COVID-19-infected alveolar organoids using western blotting.
FIG. 6 is a view illustrating the results of RNA sequencing for COVID-19-infected alveolar organoids.
FIG. 7 is a view illustrating the results of measuring the RNA expression level of an influenza virus in influenza-infected alveolar organoids at 3 hours, 24 hours, and 48 hours after injection of an influenza virus (PR8), according to the amount of influenza virus (PR8) injected into the alveolar organoids.

### [Modes of the Invention]

The present inventors treated an alveolar organoid with a respiratory virus to infect the alveolar organoid with the respiratory virus and establish a respiratory viral infection model using the alveolar organoid, thereby completing the present invention.

Hereinafter, the present invention will be described in detail.

The present invention provides a method for producing a respiratory virus-infected alveolar organoid, the method including infecting an alveolar organoid with a respiratory virus.

Further, the present invention provides a respiratory virus-infected alveolar organoid produced by the method for producing a respiratory virus-infected alveolar organoid.

As used herein, the "organoid" refers to a cell mass having a 3D conformation and refers to a reduced and simplified version of an organ prepared through an artificial culture process that is not collected or obtained from an animal, and the like. The origin of the cells which constitute it is not limited. Organoids may be derived from tissues, embryonic stem cells or induced pluripotent stem cells, and may be cultured in three dimensions due to their self-renewal and differentiation capability. In the present invention, the organoid may be an alveolar organoid produced by differentiating alveolar cells.

In the present invention, the respiratory virus may be infected so that a multiplicity of infection (MOI) is 0.001 to 10, 0.01 to 10, 0.1 to 10, 1 to 10, 5 to 10, 8 to 10, 0.001 to 8, 0.01 to 8, 0.1 to 8, 1 to 8, 3 to 8, 5 to 8, 0.001 to 5, 0.001 to 1, 0.01 to 1, or 0.1 to 1, but the MOI is not limited thereto. According to an embodiment of the present invention, specifically, when the respiratory virus is severe acute respiratory syndrome coronavirus 2 (SARS-CoV-2, COVID-19, Corona-19), the virus may be injected into the organoids at an MOI of 0.1 to 8, and when the respiratory virus is an influenza virus, the virus may be injected into the organoids at an MOI of 0.001 to 1, but the MOI is not limited thereto.

As used herein, the "multiplicity of infection (MOI)" refers to the average number of viruses used to infect a single cell.

As used herein, the "infection" means that, as a result of treating an alveolar organoid with a respiratory virus, the respiratory virus proliferates in the alveolar organoid, the respiratory virus is found in the respiratory organoid, the amount of the respiratory virus increases, or the expression of specific factors associated with the respiratory virus is confirmed. According to an embodiment of the present invention, after treating alveolar organoids with the COVID-19 virus among respiratory viruses, it was confirmed that the alveolar organoids were infected with the COVID-19 virus by confirming the expression of spike and nucleocapsid, which are specific factors of the COVID-19 virus in the alveolar organoids.

In the present invention, the respiratory virus-infected alveolar organoids may be cultured in three dimensions using Matrigel, allowing the organoids to be maintained in a form similar to that of lung tissue.

As used herein, "Matrigel" (product name of BD Biosciences) refers to a protein complex extracted from sarcoma cells of Engelbreth-Holm-Swarm (EHS) mice. Matrigel includes an extracellular matrix (ECM) such as a laminin, collagen and a heparan sulfate proteoglycan, and growth factors such as a fibroblast growth factor (FGF), an epiderma growth factor (EFG), an insulin-like growth factor (IGF), transforming growth factor-beta (TGF-β), and a platelet-derived growth factor (PDGF).

In the present invention, Matrigel may be attached to a culture plate and have a dome shape. When organoids or Matrigel including the organoids are cultured by attaching the organoids or Matrigel to a culture plate, there are various advantages in terms of culture efficiency compared to floating culture, in which the organoids float without being attached. For example, in the case of long-term culture of 5 days or more, it is essential to replace the culture medium every 2 to 3 days, and in the case of floating culture, there is a risk of losing organoids when replacing the medium, but when the organoids are attached and fixed to the culture plate, they may not be lost when replacing the medium, and the medium replacement time may be shortened.

Furthermore, due to their nature, organoids have the characteristics in that the production results vary significantly depending on the culture conditions. Examples of culture conditions include the number of days cultured, culture temperature, culture medium, and culture substrate, which are important factors that determine the characteristics of organoids, and among these, the medium condition plays the greatest role in growing organoids to have characteristics similar to those of desired tissues or organs by regulating various signaling pathways in cells or stem cells. Therefore, in the present invention, the culture of respiratory virus-infected alveolar organoids may be performed in Ham's F12 including dexamethasone, 3-isobutyl-1-methylxanthine (IBMX), B27 supplement, N2 supplement, bovine serum albumin (BSA), HEPES, CaCl₂, 8-BrcAMP, KGF, FGF-10, and antibiotic-antimycotic.

As used herein, "spike" refers to the spike glycoprotein, a protein in the form of a spike that protrudes from the viral capsid or viral envelope as viewed through an electron microscope, and is used when the virus binds to a receptor on the host cell.

As used herein, "nucleocapsid" refers to a protein structure that includes RNA or DNA, which is the genetic information of a virus, inside.

In the present specification the 'treatment' can be used interchangeably with 'addition' or 'administration.'

In the present invention, the respiratory virus may be one or more selected from the group consisting of an adenovirus, metapneumovirus, rhinovirus, parainfluenza virus, influenza virus, respiratory syncytial virus, and coronavirus, but is not limited thereto. According to an embodiment of the present invention, the respiratory virus may be an influenza virus (PR8) or severe acute respiratory syndrome coronavirus 2, but is not limited thereto.

The "influenza virus (PR8)" refers to a new strain of influenza that is a variant of H1N1, and belongs to the influenza A virus, of which there are various types and the type changes every year.

The "severe acute respiratory syndrome coronavirus 2 (SARS-CoV-2, COVID-19, Corona-19)" is a type of coronavirus 2 that was first identified in 2019, and is classified as a positive-sense single-stranded RNA virus. The disease caused by this virus was named coronavirus disease 2019, and is abbreviated as COVID-19.

According to an embodiment of the present invention, when the respiratory virus is severe acute respiratory syndrome coronavirus 2, the respiratory virus-infected alveolar organoids may have decreased expression of one or more proteins selected from the group consisting of ACE2, TMPRESS2, AT1, AT2, and SFTPC proteins compared to non-infected alveolar organoids; or

the respiratory virus-infected alveolar organoids may have increased expression of one or more proteins selected from the group consisting of IFNRα/β, STAT1, and STAT2 proteins compared to non-infected alveolar organoids, but are not limited thereto.

As used herein, the "non-infected" alveolar organoid refers to an alveolar organoid in a normal state in which no respiratory virus can be identified inside the alveolar organoid because the alveolar organoid is not infected with a respiratory virus. **In** the present invention, the "non-infected" alveolar organoids were used as a control.

**In** addition, the present invention provides a method for screening a therapeutic agent for respiratory viral infections, the method including treating the respiratory virus-infected alveolar organoid according to the present invention with a candidate substance for a therapeutic agent for respiratory viral infections.

Furthermore, the present invention provides a use of the respiratory virus-infected alveolar organoid according to the present invention for screening a therapeutic agent for respiratory viral infections.

In the present invention, the method for screening a therapeutic agent for respiratory virus infections may further include the following steps, but is not limited thereto:
(a) confirming the amount of respiratory virus in the respiratory virus-infected alveolar organoid after treatment with a candidate substance for a therapeutic agent for respiratory viral infections; and
(b) selecting the candidate substance as a therapeutic agent for respiratory virus infections when the amount of respiratory virus in the respiratory virus-infected alveolar organoid is decreased compared to before treatment with the candidate substance in step (a).

In the present invention, the "candidate substance" may be a (poly)nucleotide, DNA, RNA, an amino acid, an aptamer, a protein, a compound, a natural product, a natural extract, and the like, and is not limited thereto as long as it is a substance that can be used for the prevention or treatment of respiratory viral infections.

Throughout the specification of the present application, when one part "includes" any component, unless stated otherwise, this does not mean that another component is excluded, but means that another component may be further included. Throughout this specification, a term of degree, such as "about" or "substantially", is used in a corresponding numerical value or used as a meaning close to the numerical value when manufacturing and material tolerance errors inherent in the stated meaning are presented, and is used to prevent an unconscientious infringer from illegally using disclosed contents including a numerical value stated as an exact or absolute value in order to help understand the present invention. Throughout the specificationof the present application, the term "step ... " or "step of ..." does not mean a "step for ...".

Throughout the specification of the present application, the term "combination(s) thereof" included in the Markush type expression means a mixture or combination of at least one selected from the group consisting of constituents described in the Markush type expression, and means including at least one selected from the group consisting of the constituents.

Throughout the specification of the present application, the description "A and/or B" means "A or B, or A and B".

In cases where certain embodiments can be implemented differently, specific steps may be performed differently from the order described. For example, two steps described in succession may be performed substantially concurrently, or may be performed in reverse order to that described.

Hereinafter, preferred examples for helping to understand the present invention will be suggested. However, the following examples are provided only so that the present invention may be more easily understood, and the content of the present invention is not limited by the following examples.

### [Experimental Methods]

### 1. Production of alveolar organoids

After type II alveolar cells isolated from lung tissue were separated from a culture dish using an Animal Component-Free Cell Dissociation Kit (Cat. 05426, STEMCELL), the separated cells were put into diluted Matrigel (60% Matrigel + 40% SAGM) in an amount of 5x10³/50 µl and mixed. 50 µl of a gel including the cells was aliquoted into a culture plate, and the culture plate was incubated at 37°C in a CO₂ incubator for 30 minutes for gelation. After gelation was completed, a SAGM medium was added thereto, and the cells were cultured for 7 days (the medium was replaced with fresh medium every two days). After the cells were cultured in the SAGM medium for 7 days, the medium was changed to an alveolar differentiation medium and the cells were cultured (the medium was replaced with fresh medium every two days). The cells were cultured in the alveolar differentiation medium for at least 21 days and up to 40 days, and the produced alveolar organoids were verified through immunofluorescence staining and the like, and then used. The composition of the alveolar differentiation medium used is shown in Table 1.

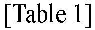

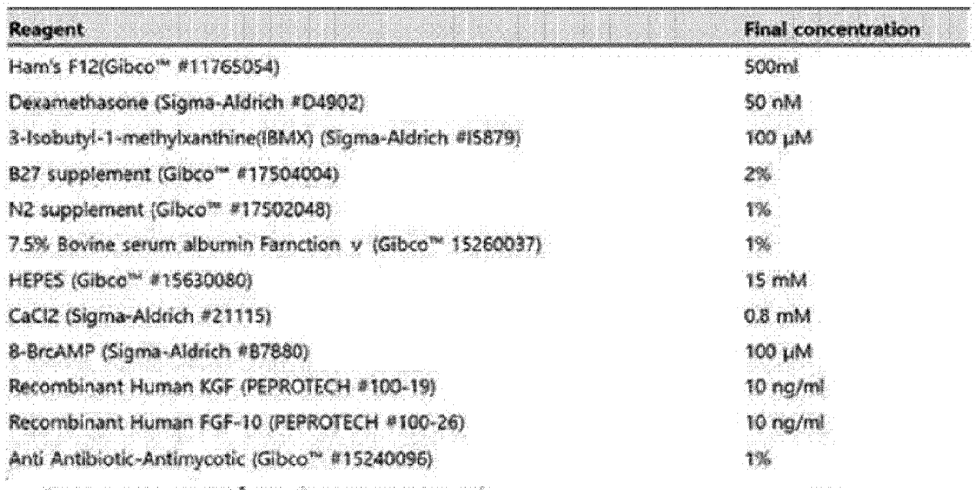

### 2. Production of COVID-19-infected alveolar organoids

Alveolar organoids produced in Matrigel were separated from the Matrigel using cold PBS. The separated alveolar organoids were infected with COVID-19 by injecting the COVID-19 virus at a multiplicity of infection (MOI) of 0.1 to 8. The COVID-19-infected alveolar organoids were incubated in the alveolar differentiation medium shown in Table 1 at 37°C for 1 hour. After 1 hour, the medium was removed by centrifugation and the cells were washed twice with PBS. Then, the alveolar organoids were transferred back to Matrigel and cultured in the form of a dome in a 48-well plate, and then cultured until sampling.

To measure COVID-19 virus levels, culture supernatants of COVID-19-infected alveolar organoids were collected to measure the amount of viral RNA, and simultaneously, cell lysates were collected from four wells to prepare samples for comparing the expression levels of the viral spike protein and nucleocapsid protein.

### 3. Production of influenza-infected alveolar organoids

Alveolar organoids were produced in the same manner as those produced by the method for producing a COVID-19-infected alveolar organoid, and instead of the COVID-19 virus, an influenza virus (PR8) was injected at a multiplicity of infection (MOI) of 0.001 to 1 to produce influenza-infected alveolar organoids.

### 4. Viral RNA quantification and TCID50 measurements

Viral RNA was purified from culture supernatants at different time points using a Viral RNA Purification Kit (Qiagen, Hilden, Germany). For one-step RT-PCR targeting the viral N gene, viral RNA quantification was performed using a diagnostic kit (PCL Inc., Seoul, Republic of Korea) and a CFX96 Touch real-time PCR instrument (Bio-Rad, Hercules, CA). In all experimental sets, RNA purified from serial dilutions of virus stocks whose plaque titers were confirmed was used as a standard to quantify viral RNA copy numbers in samples.

### [Examples]

### Example 1. Confirmation of COVID-19 infection in COVID-19-infected alveolar organoids

### 1-1. Confirmation of COVID-19 infection

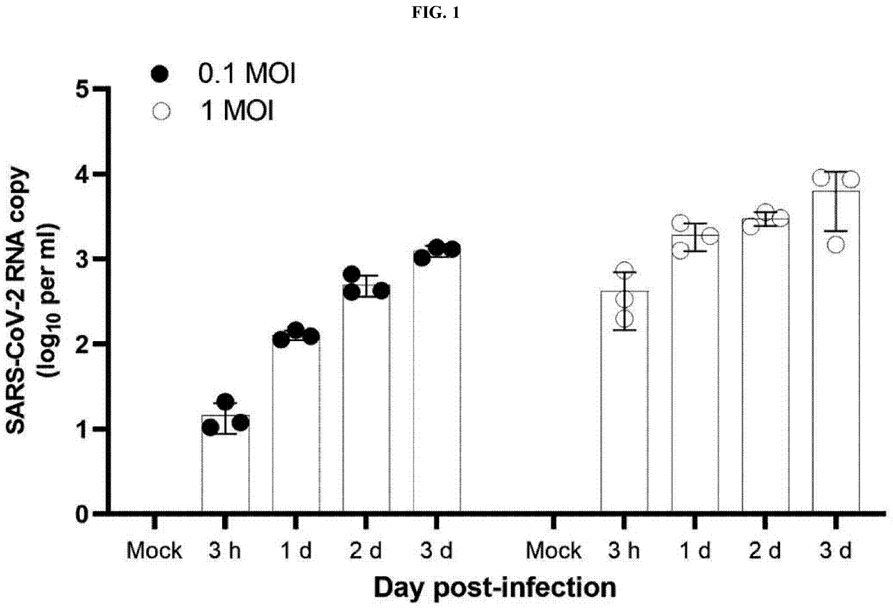

As shown in FIG. 1, to confirm whether the produced COVID-19-infected alveolar organoids were infected with COVID-19, the amount of virus released into the culture medium from the COVID-19-infected alveolar organoids was confirmed according to the amount of COVID-19 virus injected and the period after COVID-19 injection.

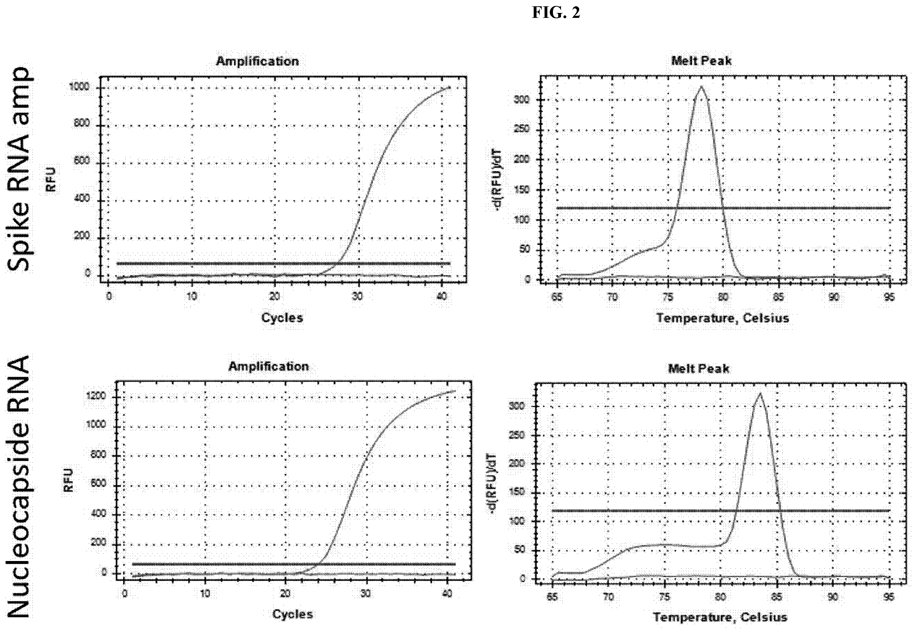

In addition, as shown in FIG. 2, by confirming the expression of spike and nucleocapsid, which are specific virus factors, in the COVID-19-infected alveolar organoids, it was confirmed that the COVID-19-infected alveolar organoids were infected with COVID-19.

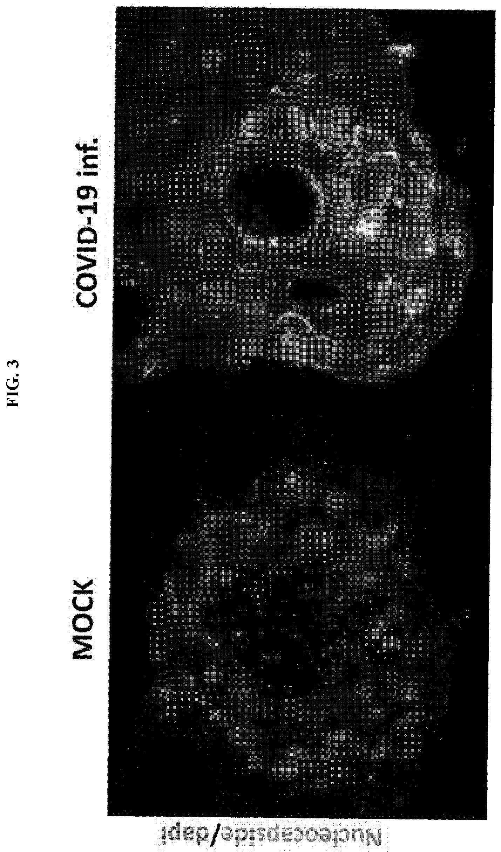

Furthermore, as shown in FIG. 3, by confirming the expression of nucleocapsid, which is a specific virus factor, in the COVID-19-infected alveolar organoids using fluorescent staining, it was confirmed that the COVID-19-infected alveolar organoids were infected with COVID-19.

### 1-2. Establishment of COVID-19 disease model using COVID-19-infected alveolar organoids

In order to establish a COVID-19 disease model using COVID-19-infected alveolar organoids, the expression of ACE2 and TMPRESS2 proteins, which are the main cell infiltration pathways of the COVID-19 virus, was confirmed in the alveolar organoids using fluorescent staining and western blotting.

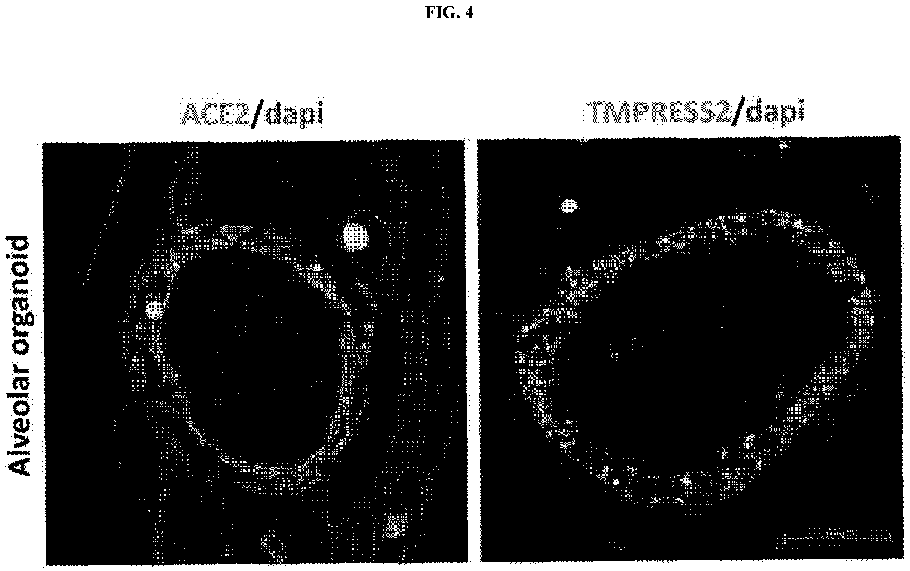

As shown in FIG. 4, as a result of fluorescent staining, ACE2 and TMPRESS2 could be identified in the COVID-19-infected alveolar organoids.

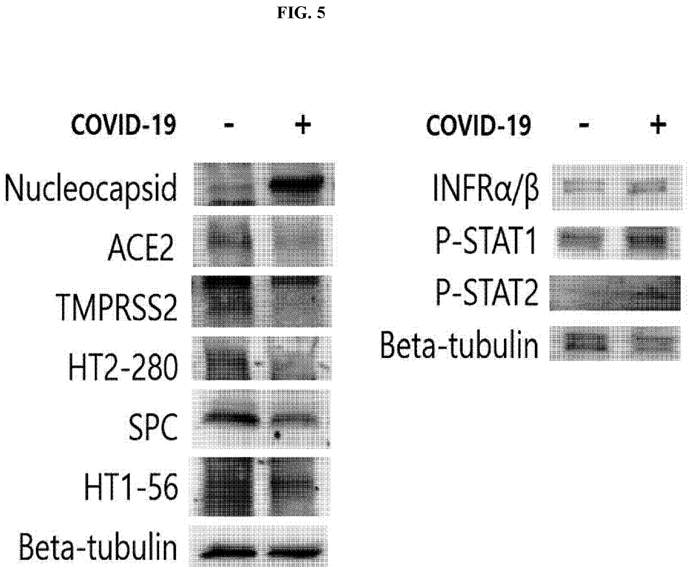

As shown in FIG. 5, when the alveolar organoids were infected with the COVID-19 virus, changes in key factors that indicate alveolar properties in the alveolar organoids were observed using western blotting. It could be confirmed that the receptors ACE2 and TMPRESS2 were decreased due to infection with COVID-19 and the expression of AT2 (HT2-280), AT1 (HT1-56) and SFTPC (SPC; surfactant protein C), which are characteristic factors of alveolar organoids, was also decreased as a whole by infection with COVID-19. In addition, it could be confirmed that infection with COVID-19 increases the expression of IFNRα/β and STAT1/2, which are receptors that indicate the signaling pathway.

### 1-3. RNA sequencing

Changes in RNA expression were confirmed in alveolar organoids upon infection with the COVID-19 virus.

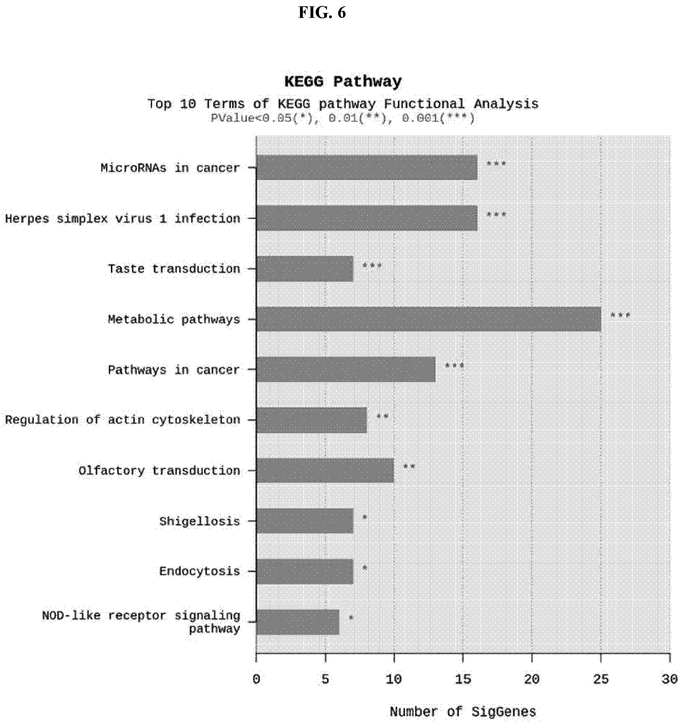

As a result, as shown in FIG. 6, it was confirmed that upon infection with the COVID-19 virus, changes in genes that regulate the expression of the cytoskeleton, which causes morphological structural changes in cells, and changes in genes that induce changes in the metabolism of host cells due to intracellular infection were noticeably observed.

### Example 2. Confirmation of influenza infection in influenza-infected alveolar organoids

To confirm whether the produced influenza-infected alveolar organoids were infected with influenza, changes in RNA expression of the influenza virus were confirmed in the influenza-infected alveolar organoids.

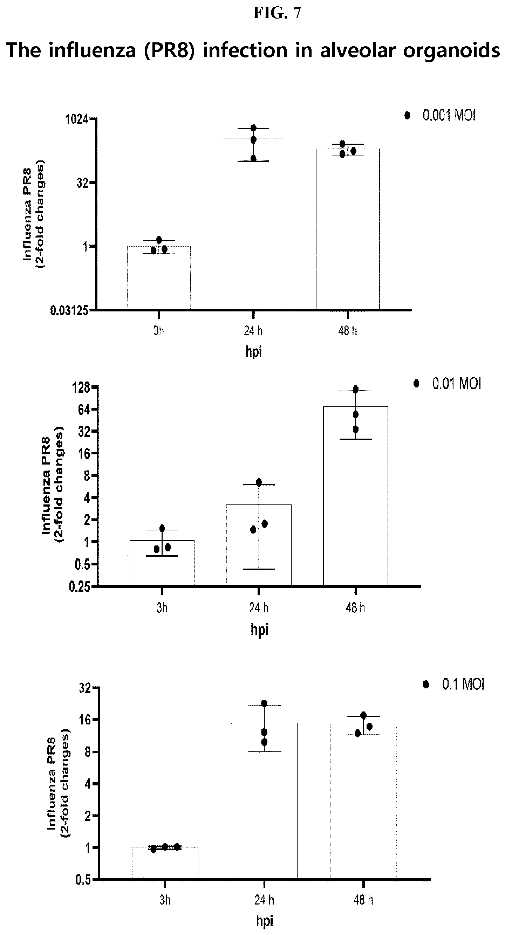

As a result, as shown in FIG. 7, it could be confirmed that the influenza virus RNA increased at 24 hours and 48 hours based on the amount of RNA expressed at 3 hours at each viral MOI.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

Respiratory virus-infected alveolar organoids produced according to the present invention can be usefully used in preclinical or clinical drug screening and the like for the development of therapeutic agents for respiratory viral infections, and thus are industrially applicable.

## Claims

1. A method for producing a respiratory virus-infected alveolar organoid, the method comprising infecting an alveolar organoid with a respiratory virus.

2. The method of claim 1, wherein the respiratory virus is infected so that a multiplicity of infection (MOI) is 0.001 to 10.

3. The method of claim 1, wherein the respiratory virus is one or more selected from the group consisting of an adenovirus, metapneumovirus, rhinovirus, parainfluenza virus, influenza virus, respiratory syncytial virus, and coronavirus.

4. The method of claim 1, wherein the respiratory virus is an influenza virus (PR8) or severe acute respiratory syndrome coronavirus 2.

5. The method of claim 4, wherein when the respiratory virus is severe acute respiratory syndrome coronavirus 2, the respiratory virus-infected alveolar organoids have decreased expression of one or more proteins selected from the group consisting of ACE2, TMPRESS2, AT1, AT2, and SFTPC proteins compared to non-infected alveolar organoids.

6. The method of claim 4, wherein when the respiratory virus is severe acute respiratory syndrome coronavirus 2, the respiratory virus-infected alveolar organoids have increased expression of one or more proteins selected from the group consisting of IFNRα/β, STAT1, and STAT2 proteins compared to non-infected alveolar organoids.

7. A respiratory virus-infected alveolar organoid produced by the method of any one of claims 1 to 6.

8. A method for screening a therapeutic agent for respiratory viral infections, the method comprising treating the respiratory virus-infected alveolar organoid according to claim 7 with a candidate substance for a therapeutic agent for respiratory viral infections.

9. A use of the respiratory virus-infected alveolar organoid according to claim 7 for screening a therapeutic agent for respiratory viral infections.
